# EUROPÄISCHE PATENTSCHRIFT

(11) **EP 1 599 211 B1**
(45) Veröffentlichungstag und Bekanntmachung des Hinweises auf die Patenterteilung: **16.02.2011**
(21) Anmeldenummer: 04716231.8
(22) Anmeldetag: 02.03.2004
(51) Int. Cl.: A61K 31/352, A61K 36/00, A61P 25/24, A61K 36/8962, A61K 36/23, A61K 36/31, A61K 36/482, A61K 36/185

(54) **VERWENDUNG VON ISORHAMNETIN ZUR BEHANDLUNG VON DEPRESSIVEN VERSTIMMUNGEN UND ERKRANKUNGEN**
USE ISOHARMNETIN FOR TREATING DEPRESSIVE STATES AND DEPRESSION
UTILISATION D'ISORHAMNETINE POUR TRAITER DES ETATS DEPRESSIFS ET LA DEPRESSION

(30) Priorität: 03.03.2003 DE 10309235; 02.04.2003 DE 10315022; 28.10.2003 DE 10350194
(43) Veröffentlichungstag der Anmeldung: 30.11.2005
(73) Patentinhaber: Dr. Willmar Schwabe GmbH & Co. KG, 76227 Karlsruhe (DE)
(72) Erfinder: CHATTERJEE, Shyam, Sunder, 76139 Karlsruhe (DE); NÖLDNER, Michael, 76139 Karlsruhe (DE); SCHÖTZ, Karl, 76448 Durmersheim (DE)
(74) Vertreter: Adam, Holger
(86) Internationale Anmeldenummer: PCT/EP2004/002085
(87) Internationale Veröffentlichungsnummer: WO 2004/078189

(56) Entgegenhaltungen:
- EP-A- 0 922 460
- DATABASE BIOSIS [Online] BIOSCIENCES INFORMATION SERVICE, PHILADELPHIA, PA, US; Juli 2002 (2002-07), NOELDER MICHAEL ET AL: "Rutin is essential for the antidepressant activity of Hypericum perforatum extracts in the forced swimming test" XP008033481 Database accession no. PREV200200468851 & PLANTA MEDICA, Bd. 68, Nr. 7, Juli 2002 (2002-07), Seiten 577-580, ISSN: 0032-0943

## Beschreibung

Depressionen gehören mit einer 1-Jahres-Prävalenzrate von ca. 10 Prozent und einer Lebens-Prävalenzrate von ca. 20 Prozent zu den häufigsten Erkrankungen in den westlichen Industrienationen. Sie beeinträchtigen den Patienten erheblich im privaten und beruflichen Bereich, irritieren seine Umgebung, belasten durch vermehrte Inanspruchnahme der ärztlichen Primärversorgung und viele Ausfalltage aufgrund von Krankschreibungen ganz enorm unser Gesundheitssystem und sind im Einzelfall sogar tödlich. Schätzungen gehen heute davon aus, dass mehr als zwei Drittel der ca. 10.000 jährlichen Suizide in Deutschland auf Depressionen zurückgehen. Depressionen werden klinisch folgendermaßen eingeteilt (H. J. Möller, Der Internist 2000, 70):
1. Endogene Depression, uni- und bipolar
2. Depressive Neurose
3. Reaktive Depression
4. Depression bei schizoaffektiver Psychose
5. Organisch bedingte Depression
6. Depressive Symptome bei Demenz

Darüber hinaus werden depressive Erkrankungen aufgrund des Ausprägungsgrades als leicht, mittelgradig oder schwer klassifiziert.

Vorstufen depressiver Erkrankungen können sich äußern in Niedergeschlagenheit, Unlustgefühl, Melancholie, Antriebsschwäche, schwankender oder eingetrübter Stimmungslage oder Einschränkungen des emotionalen Wohlbefindens.

Trotz einer Vielzahl unterschiedlicher Hypothesen sind die Ursachen depressiver Erkrankungen nur unzureichend geklärt. Zur medikamentösen Behandlung der

Depression und anderer affektiver Störungen sind z.B. die im folgenden aufgeführten Arzneimittelgruppen geeignet (H. J. Möller, Der Internist 2000, 70):
1. Selektive Serotonin-Wiederaufnahme-Hemmer (SSRI).
2. Selektive Noradrenalin-Wiederaufnahme-Hemmer (SNRI)
3. Selektive Serotonin und Noradrenalin-Wiederaufnahme-Hemmer (SSNRI).
4. Trizyklische Antidepressiva (TCA)
5. MAO-A Hemmer
6. Sonstige synthetische Präparate
7. Phytopharmaka

All den hier aufgeführten Behandlungsverfahren ist gemein, dass sie, wenn überhaupt, erst nach einer Behandlungsdauer von 10-14 Tagen wirksam werden. Bei ca. 20 % der Patienten ist die medikamentöse Therapie auch bei Kombination verschiedener Präparate unzureichend. Darüber hinaus haben alle genannten Behandlungsverfahren den Nachteil unerwünschte Nebenwirkungen zu verursachen, die häufig zum Abbruch der Therapie führen. Es besteht also ein erheblicher Bedarf, weitere Mittel zur Behandlung depressiver Erkrankungen und anderer affektiver Störungen und ihrer Vorstufen bereitzustellen, insbesondere solche Mittel, die einen oder mehrere der genannten Nachteile ganz oder teilweise überwinden.

Aufgabe der vorliegenden Erfindung ist es somit, derartige Mittel bereitzustellen.

Diese Aufgabe wird erfindungsgemäß gelöst durch die Verwendung von Isorhamnetin, eines Glykosids des Isorhamnetins oder von Pflanzenteilen und/oder von daraus hergestellten Extrakten, die Isorhamnetin in freier oder gebundener Form enthalten, zur Herstellung eines Arzneimittels bzw. diätetischen Nahrungsmittels zur Behandlung von depressiven Verstimmungen und Erkrankungen sowie von Niedergeschlagenheit, Unlustgefühlen, Melancholie, Antriebsschwäche, schwankender oder eingetrübter Stimmungslage oder zur Verbesserung des emotionalen Wohlbefindens, sowie durch ein Arzneimittel und ein diätetisches Nahrungsmittel zur Behandlung oder zur Unterstützung der Behandlung von depressiven Verstimmungen und Erkrankungen sowie von Niedergeschlagenheit, Unlustgefühlen, Melancholie, Antriebsschwäche, schwankender oder eingetrübter Stimmungslage oder zur Verbesserung des emotionalen Wohlbefindens, gekennzeichnet durch einen Gehalt an Isorhamnetin, eines Glykosids des Isorhamnetins oder von Pflanzenteilen und/oder von daraus hergestellten Extrakten, die Isorhamnetin in freier oder gebundener Form enthalten sowie durch ein Arzneimittel bzw. diätetisches Nahrungsmittel als orale Darreichungsform, die zudem geeignete pharmazeutisch verträgliche Hilfsstoffe enthält.

Isorhamnetin (3,5,7-Trihydroxy-2-(4-hydroxy-3-methoxyphenyl)-4H-1-benzopyran-4-on) gehört zur Gruppe der Flavone und kommt als Inhaltsstoff in verschiedenen Pflanzen vor. Isorhamnetin kann darüber hinaus im tierischen und menschliche Organismus durch metabolischen Umbau aus anderen Flavonen gebildet werden. Als Folge dieses Stoffwechsels können auch durch Aufnahme von Pflanzen, die selbst kein Isorhamnetin enthalten, nachweisbare Isorhamnetin-Plasmaspiegel erreicht werden. Bisher ist für Isorhamnetin keine antidepressive Wirkung gezeigt worden.

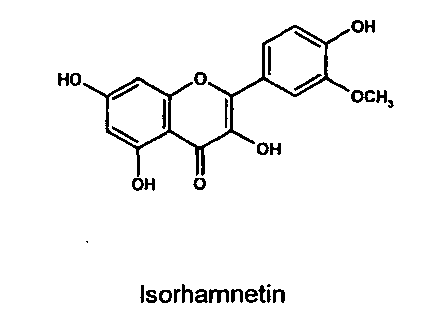

Überraschenderweise wurde festgestellt, dass Isorhamnetin, Glykoside des Isorhamnetins oder Pflanzenteile und/oder daraus hergestellte Extrakte, die mindestens einen der vorstehend genannten Stoffe in ausreichender Menge enthalten oder hydrolytisch Isorhamnetin freisetzen, im Tierversuch deutlich antidepressiv wirksam sind. Diejenigen Extrakte, die mindestens einen der genannten Stoffe in ausreichender Menge enthalten, verfügen über einen Gehalt von 0,2 % bis 100 %, bevorzugt 1 % bis 10 % an Isorhamnetin und/oder Glykosiden des Isorhamnetins, wobei der Übergang von hochangereicherten Spezialextrakten zum Reinstoff (nahe 100 %) keine Lücke aufweist.

Eine derartige Wirkung ist bisher für Isorhamnetin und die nachfolgend genannten Pflanzen und Extrakte davon nicht beschrieben, und war aufgrund der bisher bekannten pharmakologischen und klinischen Effekte nicht zu erwarten.

Beispiele für erfindungsgemäß verwendbare Pflanzen sind Allium Cepa, Anethum graveolens, Brassica oleracea, Cassia senna, Opuntia-Arten, bevorzugt Opuntia ficus-indica, Peumus boldus, Primula veris, Sambucus nigra und Selenicereus grandiflorus. Die Erfindung ist jedoch nicht auf die genannten Pflanzen beschränkt.

Die Extrakte können nach bekannten Herstellungsverfahren in variabler Zusammensetzung mit Lösungsmitteln wie z. B. Wasser, Methanol, Ethanol, Aceton, etc. und deren Gemische, bei Temperaturen von Raumtemperaturen bis 100 °C unter gelinder bis heftiger Durchmischung innerhalb von 10 Min. bis 24 Std. unter Normaldruck bis zu 200 bar erhalten werden. Zur Anreicherung von Isorhamnetin und/oder isorhamnetinbildenden Vorläufern können weitere Konzentrierungsschritte durchgeführt werden wie z. B. Flüssig-Flüssig-Verteilung mit z. B. 1-Butanol/Wasser oder Ethylacetat/Wasser, Adsorption-Desorption an lonenaustauscher, LH20, HP20 und andere Harze oder chromatographische Abtrennungen über RP18, Kieselgel, etc.

Isorhamnetin, Glykoside des Isorhamnetins oder Pflanzenteile und/oder daraus hergestellte Extrakte, die Isorhamnetin in freier oder gebundener Form enthalten, können in Form von Pulvern, Granulaten, Tabletten, Dragees oder Kapseln oder auch als Lösung vorzugsweise oral verabreicht werden.

Dazu wird Isorhamnetin, ein Glykosid des Isorhamnetins oder Pflanzenteile und/oder daraus hergestellte Extrakte, die Isorhamnetin in freier oder gebundener Form enthalten mit geeigneten pharmazeutisch verträglichen Hilfsstoffen wie z.B. Laktose, Cellulose, Siliciumdioxid, Croscarmellose und Magnesiumstearat gemischt und zu Tabletten gepresst, die gegebenenfalls mit einem geeigneten Überzug z.B. aus Hydroxymethylpropylcellulose, Polyethylenglykol, Farbstoffen (z. B. Titandioxid, Eisenoxid) und Talkum versehen werden.

Die oben genannten Stoffe können auch, ggf. unter Zusatz von Hilfsstoffen wie z.B. Stabilisatoren, Füllmittel etc., in Kapseln abgefüllt werden. Die Dosierung erfolgt dabei so, dass pro Tag 5 bis 500 mg, bevorzugt 20 bis 200 mg Rutin und/oder Isorhamnetin bzw. diejenige Menge Isorhamnetin-Derivat oder Pflanzenextrakt, das bzw. der die vorstehend genannte Isorhamnetin-Menge freisetzt bzw. enthält, zugeführt werden. Im Fall derjenigen Extrakte, die metabolisch Isorhamnetin freisetzen, wird die Dosierung so gewählt, dass ein Isorhamnetin-Blutplasmaspiegel nachgewiesen werden kann, der demjenigen nach Gabe von 5 bis 500 mg, bevorzugt 20 bis 200 mg Isorhamnetin entspricht.

Bevorzugt ist die Abfüllung der erfindungsgemäßen Stoffe zusammen mit Ölen, eine besonders bevorzugte Form umfasst die Abfüllung der Extrakte in Kapseln zusammen mit Ölen, die ungesättigte Fettsäuren, vorzugsweise ω3-Fettsäuren, enthalten, wie beispielsweise Borretschöl, Nachtkerzensamenöl, Fischöl, Johannisbeerkernöl, Leinöl oder Perillasamenöl, wodurch eine Verbesserung der Bioverfügbarkeit der wirksamkeitsbestimmenden Komponenten erreicht wird.

Die Wirksamkeit der genannten Reinstoffe, Pflanzenteile und Pflanzenextrakte bei depressiven Erkrankungen wird durch die nachstehend beschriebenen Versuche belegt.

Die antidepressive Wirksamkeit wurde mit dem sogenannten "Forced Swimming Test" an Ratten geprüft. Bei der Durchführung dieses Tests werden Ratten während einer definierten Zeit von 5 Minuten in eine für sie ausweglose Situation gebracht (wassergefüllter Glaszylinder). Die Ratten reagieren in diesem Test mit einer als Immobilisationszeit bezeichneten Starre, die als Korrelat einer Depression interpretiert wird. Werden die Ratten vor Durchführung des Tests mit antidepressiv wirksamen Arzneimitteln behandelt, verringert sich die Immobilisationszeit. Da andere Psychopharmaka wie z.B. Anxiolytika oder Neuroleptika in diesem Test unwirksam sind, ist dieses Testsystem zum Nachweis antidepressiver Wirkungen gut geeignet (Porsolt et al. 1978; Porsolt, 1991). Alle bisher bekannten Antidepressiva müssen in diesem Test, ähnlich wie beim Patienten, über einen Zeitraum von mehreren Tagen verabreicht werden um wirksam zu sein. Die Versuchstiere wurden entweder mit der Prüfsubstanz, oder zu Kontrollzwecken nur mit dem Lösungsmittel oder zum Vergleich der Effektivität mit dem Tricyklischen Antidepressivum Imipramin behandelt. Imipramin wurde als Standard Vergleichssubstanz gewählt, weil es sowohl in der psychiatrischen Praxis, als auch im tierexperimentellen Modell eines der am stärksten wirkenden Antidepressiva ist.

Die folgenden Tabellen zeigen beispielhaft die Wirksamkeit von Isorhamnetin und Pflanzenextrakten, die Glykoside des Isorhamnetins in ausreichenden Mengen enthalten. Die Hemmung der Immobilität wurde in allen Tabellen zu Vergleichszwecken in Prozent - Hemmung gegen die Kontrollgruppe ausgedrückt.

**Tabelle 1**

| **Substanz** | **Dosis** | **Hemmung der Immobilität** |
|---|---|---|
| | **[mg/kg]** | **[% der Kontrolle]** |
| Isorhamnetin | 3 | 17 |
| Isorhamnetin | 10 | 56 |
| Imipramin | 3 | 3 |
| Imipramin | 10 | 24 |

**Tabelle 2**

| **Substanz** | **Dosis** | **Hemmung** | **Isorhamnetin** |
|---|---|---|---|
| | **[mg/kg]** | **[% der Kontrolle]** | **[mg/kg]** (bestimmt nach Hydrolyse) |
| S. grandiflorus-Extrakt (Beispiel 1) | 3 | 13 | 0,11 |
| S. grandiflorus-Extrakt (Beispiel 1) | 10 | 30 | 0,36 |
| S. grandiflorus-Extrakt (Beispiel 1) | 30 | 38 | 1,08 |
| S. grandiflorus-Extrakt (Beispiel 1) | 100 | 48 | 3,60 |
| Imipramin | 20 | 43 | |

**Tabelle 3**

| **Substanz** | **Dosis** | **Hemmung** | **Isorhamnetin** |
|---|---|---|---|
| | **[mg/kg]** | **[% der Kontrolle]** | **[mg/kg]** (bestimmt nach Hydrolyse) |
| Opuntiaextrakt (Beispiel 2) | 10 | 16 | 0,44 |
| Opuntiaextrakt (Beispiel 2) | 30 | 34 | 1,32 |
| Opuntiaextrakt (Beispiel 2) | 100 | 52 | 4,40 |
| Opuntiaextrakt (Beispiel 2) | 300 | 83 | 13,20 |
| Imipramin | 30 | 64 | |

### Beispiele

### Beispiel 1: Extrakt aus Selenicereus grandiflorus

200 g fein gemahlene Droge werden zweimal mit je 1400 g 60 Gw.-% EtOH jeweils 1 Std. bei 60°C gerührt, anschließend die Suspension über eine Fritte P4 abgesaugt, die vereinigten Filtrate im Vakuum bei 40 °C vom EtOH befreit, der verbliebene wässrige Rückstand eingefroren und lyophilisiert. Der erhaltene Feststoff wird im Vakuum bei 40°C über P₂O₅ und KOH getrocknet: 30,2 g (15.1 %) Extrakt mit 0,1 % Isorhamnetin (bestimmt ohne Hydrolyse) bzw. 3,6 % Isorhamnetin (bestimmt nach Hydrolyse).

### Beispiel 2: Extrakt aus Blüten von Opuntia ficus-indica

2000 g fein gemahlene Droge werden 2 mal mit je 14 kg 60 Gew. % EtOH jeweils 1 Std. bei 60°C gerührt. Anschließend wird die Suspension über eine Filternutsche abgesaugt, die vereinigten Filtrate im Vakuum bei erhöhter Temperatur vom wässrigen Ethanol befreit und das verbliebene Öl im Vakuum bei 50°C getrocknet: 285 g (14,2%) Extrakt mit 4,4 % Isorhamnetin (bestimmt nach Hydrolyse).

### Beispiel 3: Tabletten

Ein Extrakt aus Selenicereus grandiflorus wird mit Hilfstoffen gemischt und zu Tabletten verpresst (Tablettenkern = Position 1 - 6). Die Tabletten werden mit einem Überzug aus Hydroxypropylmethylcellulose versehen (Position 7 -10).

| | Bestandteil | mg/Tablette |
|---|---|---|
| 1 | Extrakt aus Selenicereus grandiflorus | 100,0 |
| 2 | Mikrokristalline Cellulose | 117,0 |
| 3 | Laktose Monohydrat | 58,0 |
| 4 | Croscarmellose | 15,0 |
| 5 | Hochdisperses Siliciumdioxid | 3,0 |
| 6 | Magnesiumstearat | 6,0 |
| 7 | Hydroxypropylmethylcellulose | 15,0 |
| 8 | Polyethylenglycol | 3,0 |
| 9 | Talkum | 1,0 |
| 10 | Titandioxid | 2,0 |

### Beispiel 4: Kapseln

Ein Extrakt aus Selenicereus grandiflorus wird mit Perillasamenöl gemischt und die erhaltene fließfähige Suspension wird mit einem geeigneten an sich bekannten Verfahren in Kapseln abgefüllt.

| | Bestandteil | mg/Kapseffüllung |
|---|---|---|
| 1 | Extrakt aus Selenicereus grandiflorus | 100,0 |
| 2 | Perillasamenöl | 450,0 |

## Patentansprüche

1. Verwendung von Isorhamnetin, eines Glykosids des Isorhamnetins oder von Pflanzenteilen und/oder daraus hergestellter Extrakte, die Isorhamnetin in freier oder gebundener Form enthalten, zur Herstellung eines Arzneimittels zur Behandlung von depressiven Verstimmungen und Erkrankungen sowie von Niedergeschlagenheit, Unlustgefühlen, Melancholie, Antriebsschwäche, schwankender oder eingetrübter Stimmungslage oder zur Verbesserung des emotionalen Wohlbefindens.

2. Verwendung von Isorhamnetin, eines Glykosids des Isorhamnetins oder von Pflanzenteilen und/oder daraus hergestellter Extrakte, die Isorhamnetin in freier oder gebundener Form enthalten, zur Herstellung eines diätetischen Nahrungsmittels zur Behandlung oder zur Unterstützung der Behandlung von depressiven Verstimmungen und Erkrankungen sowie von Niedergeschlagenheit, Unlustgefühlen, Melancholie, Antriebsschwäche, schwankender oder eingetrübter Stimmungslage oder zur Verbesserung des emotionalen Wohlbefindens.

3. Verwendung nach Anspruch 1 oder 2, wobei Isorhamnetin durch hydrolytische Spaltung eines Glykosids des Isorhamnetins entsteht.

4. Verwendung nach einem der Ansprüche 1 bis 3, wobei die Pflanzenteile und/oder die Extrakte ausgewählt sind aus den Pflanzen und/oder einem Extrakt von Allium Cepa, Anethum graveolens, Brassica oleracea, Cassia senna, Opuntia-Arten, Peumus boldus, Primula veris, Sambucus nigra oder Selenicereus grandiflorus.

5. Verwendung nach Anspruch 4, wobei die Pflanzenteile und/oder die Extrakte aus den Blüten von Selenecereus grandiflorus hergestellt werden.

6. Verwendung nach Anspruch 4, wobei die Pflanzenteile und/oder die Extrakte aus den Blüten von Opuntia ficus-indica hergestellt werden.

7. Verwendung nach einem der Ansprüche 1 bis 6, wobei zusätzlich ein Öl verwendet wird, das ungesättigte Fettsäuren enthält.

8. Verwendung nach Anspruch 7, wobei das Öl ausgewählt ist aus der Gruppe umfassend Borretschöl, Nachtkerzensamenöl, Johannisbeerkernöl, Fischöl, Leinöl und Perillasamenöl.

9. Arzneimittel zur Verwendung in der Behandlung von depressiven Verstimmungen und Erkrankungen sowie von Niedergeschlagenheit, Unlustgefühlen, Melancholie, Antriebsschwäche, schwankender oder eingetrübter Stimmungslage oder zur Verbesserung des emotionalen Wohlbefindens, **gekennzeichnet durch** einen Gehalt an Isorhamnetin, eines Glykosids des Isorhamnetins oder Pflanzenteilen und/oder daraus hergestellter Extrakte, die Isorhamnetin in freier oder gebundener Form enthalten.

10. Diätetisches Nahrungsmittel zur Verwendung in der Behandlung oder zur Unterstützung der Behandlung von depressiven Verstimmungen und Erkrankungen sowie von Niedergeschlagenheit, Unlustgefühlen, Melancholie, Antriebsschwäche, schwankender oder eingetrübter Stimmungslage oder zur Verbesserung des emotionalen Wohlbefindens, **gekennzeichnet durch** einen Gehalt an Isorhamnetin, eines Glykosids des Isorhamnetins oder Pflanzenteilen und/oder daraus hergestellter Extrakte, die Isorhamnetin in freier oder gebundener Form enthalten.

11. Arzneimittel oder diätetisches Nahrungsmittel zur Verwendung nach Anspruch 9 oder 10, des Weiteren ein Öl enthaltend, das ungesättigte Fettsäuren enthält.

12. Arzneimittel oder diätetisches Nahrungsmittel zur Verwendung nach Anspruch 11, wobei das Öl ausgewählt ist aus der Gruppe umfassend Borretschöl, Nachtkerzensamenöl, Johannisbeerkernöl, Fischöl, Leinöl und Perillasamenöl.

13. Arzneimittel oder diätetisches Nahrungsmittel zur Verwendung nach einem der Ansprüche 9 bis 12 als orale Darreichungsform.

## Claims

1. Use of isorhamnetin, a glycoside of isorhamnetin, or of plant parts and/or extracts prepared thereof which contain isorhamnetin in free or bound form for the preparation of a medicament for the treatment of depressive disorders and diseases as well as despondency, listlessness, melancholy, lack of drive, fluctuating or bleak mood, or for improvement of emotional well-being.

2. Use of isorhamnetin, a glycoside of isorhamnetin, or of plant parts and/or extracts prepared thereof which contain isorhamnetin in free or bound form for the preparation of a dietetic food for the treatment or support of treatment of depressive disorders and diseases as well as despondency, listlessness, melancholy, lack of drive, fluctuating or bleak mood, or for improvement of emotional well-being.

3. Use of claim 1 or 2, wherein isorhamnetin is formed by hydrolytic cleavage of a glycoside of isorhamnetin.

4. Use according to one of claims 1 to 3, wherein the plant parts and/or the extracts are selected from the plants and/or an extract of Allium cepa, Anethum graveolens, Brassica oleracea, Cassia senna, Opuntia species, Peumus boldus, Primula veris, Sambucus nigra or Selenicereus grandiflorus.

5. Use according to claim 4, wherein the plant parts and/or extracts are prepared from the flowers of Selenecereus grandiflorus.

6. Use according to claim 4, wherein the plant parts and/or extracts are prepared from the flowers of Opuntia ficus-indica.

7. Use according to one of claims 1 to 6, wherein an oil containing unsaturated fatty acids is used in addition.

8. The use according to claim 7, wherein the oil is selected from the group consisting of borage oil, evening primrose seed oil, black currant seed oil, fish oil, linseed oil and perilla seed oil.

9. Medicament for use in the treatment of depressive disorders and diseases as well as despondency, listlessness, melancholy, lack of drive, fluctuating or bleak mood, or for improvement of emotional well-being, **characterized by** a content of isorhamnetin, a glycoside of isorhamnetin or plant parts and/or extracts prepared thereof, which comprise isorhamnetin in free or bound form.

10. Dietetic food for use in the treatment or for supporting the treatment of depressive disorders and diseases as well as despondency, listlessness, melancholy, lack of drive, fluctuating or bleak mood, or for improvement of emotional well-being, **characterized by** a content of isorhamnetin, a glycoside of isorhamnetin or plant parts and/or extracts prepared thereof, which comprise isorhamnetin in free or bound form.

11. Medicament or dietetic food for use according to claim 9 or 10, further comprising an oil comprising unsaturated fatty acids.

12. Medicament or dietetic food for use according to claim 11, wherein the oil is selected from the group consisting of borage oil, evening primrose seed oil, black currant seed oil, fish oil, linseed oil and perilla seed oil.

13. Medicament or dietetic food for use according to one of claims 9 to 12 as an oral administration form.

## Revendications

1. Utilisation d'isorhamnétine, d'un glycoside de l'isorhamnétine ou de parties de plantes et/ou d'extraits fabriqués à partir de celles-ci, qui contiennent de l'isorhamnétine sous forme libre ou lisée, pour la fabrication d'un médicament pour le traitement d'humeurs et de pathologies dépressives ainsi que de l'abattement, des sentiments de tristesse, de la mélancolie, du manque d'entrain, d'une humeur changeante ou perturbée ou pour l'amélioration du bien-être émotionnel.

2. Utilisation d'isorhamnétine, d'un glycoside de l'isorhamnétine ou de parties de plantes et/ou d'extraits fabriqués à partir de celles-ci, qui contiennent de l'isorhamnétine sous forme libre ou liée, pour la fabrication d'un aliment diététique pour le traitement ou pour le renfort du traitement d'humeurs et de pathologies dépressives ainsi que de l'abattement, des sentiments de tristesse, de la mélancolie, du manque d'entrain, d'une humeur changeante ou perturbée ou pour l'amélioration du bien-être émotionnel.

3. Utilisation selon la revendication 1 ou la revendication 2, dans laquelle l'isorhamnétine est produite par division hydrolytique d'un glycoside de l'isorhamnétine.

4. Utilisation selon l'une des revendications 1 à 3, dans laquelle les parties de plante et/ou les extraits sont choisis parmi les plantes et/ou un extrait d*'Allium cepa,* d*'Anethum graveolens,* de *Brassica oleracea,* de *Cassia senna,* d*'Opuntia-Arten,* de *Peumus Boldus,* de *Primula veris,* de *Sambucus nigra* ou de *Selenicereus grandiflorus.*

5. Utilisation selon la revendication 4, dans laquelle les parties de plantes et/ou les extraits sont fabriqués à partir des fleurs de *Selenicereus grandiflorus.*

6. Utilisation selon la revendication 4, dans laquelle les parties de plantes et/ou les extraits sont fabriqués à partir des fleurs d*'Opuntia ficus-indica.*

7. Utilisation selon l'une des revendications 1 à 6, dans laquelle en outre une huile est utilisée qui contient des acides gras insaturés.

8. Utilisation selon la revendication 7, dans laquelle l'huile est choisie dans le groupe comprenant l'huile de bourrache, l'huile d'onagre, l'huile de pépins de groseille, l'huile de poisson, l'huile de lin et l'huile de graines de perilla.

9. Médicament à utiliser dans le traitement d'humeurs et de pathologies dépressives ainsi que de l'abattement, des sentiments de tristesse, de la mélancolie, du manque d'entrain, d'une humeur changeante ou perturbée ou pour l'amélioration du bien-être émotionnel, **caractérisé par** une teneur en isorhamnétine, un glycoside de l'isorhamnétine ou des parties de plantes et/ou des extraits fabriqués à partir de celles-ci, qui contiennent de l'isorhamnétine sous forme libre ou liée.

10. Aliment diététique à utiliser dans le traitement ou pour le renfort du traitement d'humeurs et de pathologies dépressives ainsi que de l'abattement, des sentiments de tristesse, de la mélancolie, du manque d'entrain, d'une humeur changeante ou perturbée ou pour l'amélioration du bien-être émotionnel, **caractérisé par** une teneur en isorhamnétine, un glycoside de l'isorhamnétine ou des parties de plantes et/ou des extraits fabriqués à partir de celles-ci, qui contiennent de l'isorhamnétine sous forme libre ou liée.

11. Médicament ou aliment diététique à utiliser selon la revendication 9 ou 10, contenant en outre une huile qui contient des acides gras insaturés.

12. Médicament ou aliment diététique à utiliser selon la revendication 11, l'huile étant choisie dans le groupe incluant l'huile de bourrache, l'huile d'onagre, l'huile de pépins de groseille, l'huile de poisson, l'huile de lin et l'huile de graines de perilla.

13. Médicament ou aliment diététique à utiliser selon l'une des revendications 9 à 12 en forme d'administration par voie orale.
